# EUROPEAN PATENT APPLICATION

(11) **EP 2 977 051 A1**
(43) Date of publication of application: **27.01.2016**
(21) Application number: 13876116.8
(22) Date of filing: 01.03.2013
(51) Int. Cl.: A61K 31/704, A61K 36/47, A61P 25/00, A61P 43/00, A61K 8/63

(54) **GINSENOSIDE COMPOSITION**

(71) Applicant: Kim's Korean Ginseng Co., Ltd., Kyoto-shi, Kyoto 600-8351 (JP)
(72) Inventor: KIM CHANG SOO, Kyoto-shi Kyoto 603-8324 (JP); KIM KYUNG KWANG, Kyoto-shi Kyoto 600-8351 (JP); HASEGAWA, Masayasu, Kyoto-shi Kyoto 600-8351 (JP)
(74) Representative: TBK
(86) International application number: PCT/JP2013/055600
(87) International publication number: WO 2014/132430

(57) **Abstract**

The object of the present invention is to provide a ginsenoside composition which can obtain specific and excellent result in terms of efficacy, to various symptoms and diseases.

The ginsenoside composition includes 3 components of Rb 1, Rg 3 (S), and Rg 2(S), which are ginsenosides, as an essential component. In solid base of the composition, the ginsenoside composition satisfies the following 3 requirements. That is, (Requirement 1): a content of the Rb 1 is 7.3 mg/g or more, (Requirement 2): a ratio of a content of the Rb 1 and a content of the Rg 3 (S), "Rb 1 / Rg 3 (S)", is in a range of 1.2 to 3.7, and (Requirement 3): a ratio of a content of the Rg 3 (S) and a content of the Rg 2 (S), "Rb 3 (S) / Rg 2 (S)", is in a range of 1.7 to 4.4.

## Description

### BACKGROUND OF THE INVENTION

### Field of the Invention

The present invention relates to a ginsenoside composition which is derived from a ginseng and useful for the improvement of symptoms such as indefinite complaint.

### Background Art

### (Indefinite complaint)

The indefinite complaint is the symptom that cannot be found the causative disease by the inspection although there are subjective symptoms that is vaguely bad physical condition. Further, the indefinite complaint refers to the symptom there is no effective treatment by modern medicine.

The typical symptoms of the indefinite complaint are, fatigue, loss of appetite, insomnia, cold hands and feet, bowel movement disorders, lack of motivation fullness, low back pain, stiff neck, stomach pain, abdominal bloating, sweating, muscle and joint stiffness, etc.

### (Ginseng, especially Korean ginseng)

### -1-

Recently, the effect or efficacy of the ginseng has been attracting attention. In those, Korean ginseng which indicates the improvement of symptoms of the indefinite complaint above is spotlighted. It can be said that Korean ginseng is expected in worldwide as a representative of a complementary and alternative medicine.

### -2-

Korean ginseng, which is the representative of ginsengs, is called Panax ginseng C.A. Meyer in an academic name. It has been considered that saponins contained in a root of Korean ginseng are an essence of effective components. In addition, all saponins contained in ginsengs are called "ginsenoside".

### -3-

However, Korean ginseng is a very valuable plant since difficulty of its cultivation, so that is very expensive. Therefore, it has been strongly expected that a Korean ginseng product, which can exert the maximum effect even small amounts ingestion, has been developed.

### (Ginsenoside focused in the present invention)

### -1-

In these situations, the present inventors carried out earnest works and found out the followings to complete the present invention. That is, in the ginsenosides currently discovered more than 40, when a few kinds of specified ginsenoside are used in combination in the specified amount ratio, (a) the effects to diseases (efficacy), such as indefinite complaint, remarkably improve, and (b) the width of the kinds of disease remarkably extends.

### -2-

In the present invention, as described in detail later, in various kinds of ginsenoside, "ginsenoside Rb 1" , "ginsenoside Rg 3 (especially its S-isomer)", and "ginsenoside Rg 2 (especially its S-isomer)" are focused, so that these 3 kinds of ginsenoside are mainly described in the followings.

### (Diol-based ginsenoside and triol-based ginsenoside)

The ginsenosides contained in a ginseng are a diol-based ginsenoside, a triol-based ginsenoside, and a small amount of oleanol-based ginsenoside.

Generally, it is said that the diol-based ginsenodide stimulates the parasympathetic nerves of autonomic nerves, and acts on the sedation of nerves.

Conversely, it is said that the triol-based ginsenoside stimulates the sympathetic nerves, and prompts excitement of nerves.

Therefore, It can be said that the adjustment of the physical condition is delicately changed in the balance of the diol-based ginsenoside and the triol-based ginsenoside.

### (About Rb 1)

Since the ginsenoside Rb 1, which is one of diol-based ginsenoside, is a representative ginsenoside existing more in a raw root of Korean ginseng, it has been called a major ginsenoside.

Rb 1 hardly enters in blood vessels and absorbent is low in the human body. However, it is considered that Rb 1 has an effect to suppress to absorb fat in an intestinal tract.

In addition, it is considered that Rb 1 changes to various other ginsenoside by receiving decomposition of sugar chains by intestinal microbial, so that the absorbent for the human body increases and thus the conversion products exert effects in the human body. Namely, Rb 1 acts as "pro-drug, i.e., precursor components".

### (About Rg 3 (S))

The ginsenoside Rg 3 is contained only a little in a raw root of Korean ginseng, so that it is called a minor ginsenoside. In Rg 3, there are optical isomers, i.e., S-isomer and R-isomer. In these isomers, S-isomer is a component focused in the present invention. The S-isomer is described as Rg 3 (S) below.

Rg 3 (S) is one of the ginsenoside produced by cutting sugar chains of the major ginsenoside of the diol-based ginsenoside, which are Rb 1, Rb 2, and Rc and so on.

The content of Rg 3 (S) is extremely little in raw Korean ginseng and increased by heat treatments or fermentation process (including intestinal microbial) of Korean ginseng or its extracts.

It has been known that Rg 3 (S) has good absorbency to the human body and is known as the ginsenoside having extremely high medical efficacy.

In addition, in Rg 3, it is said that the S-isomer has higher medical efficacy than the R-isomer.

### (About Rg 2 (S))

On the other hand, ginsenoside Rg 2 also has been called the minor ginsenoside and has S-isomer and R-isomer as the optical isomers. In these isomers, the S-isomer is the component focused in the present invention. The S-isomer is described as Rg 2 (S) below.

Rg 2 (S) is one of the ginsenoside produced by cutting sugar chains of Re which is the major ginsenoside of the triol-based ginsenoside.

In the ginsenoside Rg 2 (S), the content is extremely little in raw Korean ginseng and increased by heat treatments or fermentation process (including intestinal microbial) of Korean ginseng or its extracts.

It has been known that Rg 2 (S) has good absorbent in the human body and is known as the ginsenoside having extremely high medical efficacy.

In addition, in Rg 2, it is said that the S-isomer has higher medical efficacy than the R-isomer.

### (Summary of each component of ginsenoside)

The summary of the above description is the following Table 1. The arrow mark in Table 1 indicates "conversion".

**[Table 1]**

| [Diol-based ginsenoside] | | |
|---|---|---|
| Major ginsenoside | | Minor ginsenoside |
| Rb 1 | → | Rg 3 (S-isomer and R-isomer) |
| Rb 2 | → | Rg 3 (S-isomer and R-isomer) |
| Rc | → | Rg 3 (S-isomer and R-isomer) |

| [Triol-based ginsenoside] | | |
|---|---|---|
| Major ginsenoside | | Minor ginsenoside |
| Re | → | Rg 2 (S-isomer and R-isomer) |

### (Patent document 1)

In claim 1 of PCT Japanese Translation Patent Publication No. 2007-520418 (Patent document 1), the following production method of ginseng formulation.

That is,
the method comprising,
adding vinegar having pH 2 to 4 to a ginseng or a ginseng extract,
heating and extracting the mixture for 0.2 to 24 hours,
producing a ginseng formulation,
wherein a content of Rg 3 is 5 to 100% with respect to a sum of Rb 1, Rb 2, Rc, Rd, Re, Rf, Rg 1, and Rg 3, and
wherein a content of sum of Rg 3, Rg 5, and Rh 1 is 1-15%.

In claims 13 to 15, ginseng formulations having the similar components are described.

### (Patent document 2)

In PCT Japanese Translation Patent Publication No. 2009-536941 (Patent document 2), in the abstract, a production method of a black ginseng, a concentrated solution of a black ginseng, and honey pickled of a black ginseng are described. In the production method, after 9 times steaming processes, a ripening process is immediately carried out without a drying process.

Claims 1 to 4 relate to the production method of the black ginseng, and claims 5 to 6 relate to the production method of the concentrated solution of the black ginseng.

In claim 7, "the production method of the concentrated solution of the black ginseng according to claim 5 or claim 6, wherein the concentrated solution of the black ginseng contains an effective saponin component of 80 to 300 mg/g, and wherein the effective saponin includes Rg 1, Rg 2, Rg 3, Rd, Re, Rf, Rb 1, Rb 2,and Rc" is described. In Table 1 in paragraph 0067, there is the similar description, but there is no description about the amount of each component.

### (Patent document 3)

In claim 8 in PCT Japanese Translation Patent Publication No. 2008-502711 (Patent document 3), "A composition including ( i ) Rb 1, Rb 2, Rc, Rd, Re, Rf and Rg 1 and ( ii ) Rg 3, Rg 5, and Rk 1, wherein the content ratio of ( i ) / ( ii ) is (0.1 to 10) / (0.1 to 10)" is described. In claim 9, the above content ratio is limited on (1) / (0.1 to 10).

### (Patent document 4)

In claim 1 in PCT Japanese Translation Patent Publication No. 1999-501322 (Patent document 4), "A processed ginseng or a processed ginseng extract, wherein a ratio of (Rg 3 + Rg 5) / (Rc + Rd + Rb 1 + Rb 2) in ginsenoside is 1.0 or more" is described.

### (Patent document 5)

In PCT Japanese Translation Patent Publication No. 2007-529431 (Patent document 5), a traditional Chinese medicine preparation for cardiovascular and cerebrovascular diseases, in which two compounds (especially isolithospermic acid A and B) are included, is described.

In the tables in paragraphs 0075 and 0076 according to "a pill", the analytical method of "Rb 1, Rg 3 (20 R and 20 S), and Rg 2" is described.

### PRIOR ART DOCUMENTS

### Patent Documents

Patent document 1:
   PCT Japanese Translation Patent Publication No. 2007-520418
Patent document 2:
   PCT Japanese Translation Patent Publication No. 2009-536941
Patent document 3:
   PCT Japanese Translation Patent Publication No. 2008-502711
Patent document 4:
   PCT Japanese Translation Patent Publication No. 1999-501322
Patent document 5:
   PCT Japanese Translation Patent Publication No. 2007-529431

### SUMMARY OF THE INVENTION

### Problems to be Solved by the Invention

### (Ginsenoside components in patent documents 1 to 5)

Since there are descriptions of each component of ginsenoside in the above patent documents 1 to 5, the summery is described in the following Table 2. For reference, essential components in the present invention are also described.

**[Table 2]**

| Present invention | Patent document 1 | Patent document 2 | Patent document 3 | Patent document 4 | Patent document 5 |
|---|---|---|---|---|---|
| Rb1 | Rb1 | Rb1 | Rb1 | Rb1 | Rb1 |
| | Rb2 | Rb2 | Rb2 | Rb2 | |
| | Rc | Rc | Rc | Rc | |
| | Rd | Rd | Rd | Rd | Rd |
| | Re | Re | Re | | Re |
| | Rf | Rf | Rf | | Rf |
| | Rg1 | Rg1 | Rg1 | | Rg1 |
| Rg2(S) | Rg2 | | | | Rg2 |
| Rg2(R) | | | | | |
| Rg3(S) | Rg3 | Rg3 | Rg3(S) | Rg3 | Rg3(S) |
| Rg3(R) | | | Rg3(R) | | Rg3(R) |
| | Rg5 | | Rg5 | Rg5 | Rg5 |
| | Rh1 | | | | Rh1 |
| | | | | | Rh4 |
| | Rk1 | Rk1 | | | Rk1 |
| | | | | | Rk3 |

### -1-

As described in the above Table, in these patent documents 1 to 5, there is no description about setting a ratio of a content of Rg 3 (S) and a content of Rg 2 (S), "Rg3 (S) / Rg2 (S)", to be a specified range. On other words, there is no description to even focus the S-isomer, which is the optical isomer of Rg 3, and the S-isomer, which is the optical isomer of Rg 2.
In claim 6 in the patent document 5, although there is a description about Rb 1, Rg 2, and Rg 3 (20 (S) and 20 (R), i.e., (S) and (R)), there is no description about the ratio of "Rg 3 (S) / Rg 2 (S)".

### -2-

Therefore, in any of the patent documents 1 to 5, it has not been considered at all that the ratio of "Rg 3 (S) / Rg 2 (S)" is in "1.7 to 4.4" which is the case in the present invention.
In addition, in any of the patent documents 1 to 5, about the relation to "Rb1 ", it has not been considered that an excellent effect can be obtained only when a ratio of "Rb 1" to "Rg 3 (S)" and further a ratio of "Rb 1" to "Rg 3 (S) / Rg 2 (S)" are in the specified range which is the case in present invention.

### (Relationship to commercial ginsenoside preparations)

### -1-

About products of commercial ginsenoside preparations (health supplements), these components or compositions have not been almost described.

### -2-

In the process reaching to the present invention, the present inventors have been carries out for 2 years or more to analysis the contents of each component of many ginsenoside products, which are 21 commercialized products, where the components are (Rb 1, Rg 3 (Rg 3 (S) and Rg 3 (R)), Rg 2 (Rg2 (S) and Rg2 (R)), and the like. Thus, they also has been carried out to collate these data.

However, there are not commercialized ginsenoside products which satisfy the below description (a), (b), and (c) which are focused by the present inventors.
(a) A content of Rb 1 is 7.3 mg/g or more.
(b) A ratio of a content of Rb 1 (mg/g) and a content of a Rg 3 (S) (mg/g), i.e., "Rb 1/Rg 3 (S)" is 1.2 to 3.7.
(c) A ratio of a content of Rg3 (S) (mg/g) and a content of Rg 3 (S) (mg/g), i.e., "Rg 3 (S) / Rg 2 (S), is 1.7 to 4.4.

### -3-

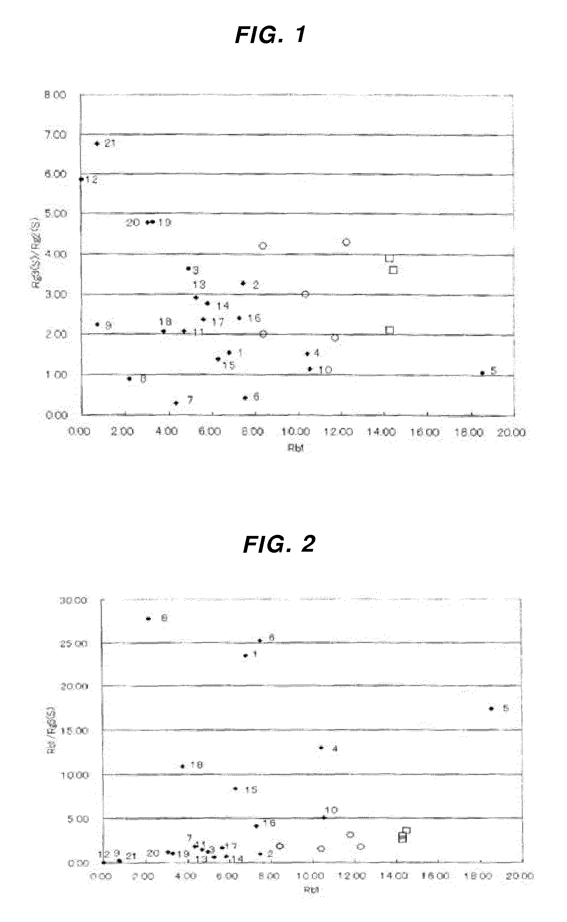

In Fig. 1, the relationship between the "content of Rb1" and the "ratio of Rb1/Rg3 (S)" of the commercial ginsenoside preparation is shown. Plots ● correspond to the above commercial ginsenoside preparations 1 to 21. In addition plots of Oand □ in Fig. 1 correspond to the exemplary embodiments of the present invention. (In five ○, two ○ are overlapped.)

### -4-

In Fig. 2, about the commercial ginsenoside preparations, the horizontal axis indicates "content of Rb 1" and the vertical axis indicates "ratio of Rg 3 (S) / Rg 2 (S)". That is, in the horizontal axis and the vertical axis, the relationship of "content of Rb 1" and "ratio of Rg 3 (S) / Rg 2 (S)" is indicated. Plots ● correspond to the above commercial ginsenoside preparations 1 to 21. In addition, plots ○ and □ in Fig. 2 correspond to the exemplary embodiments of the present invention.

### -1-

### (Object of the invention)

Under these backgrounds, the present inventors have been carried out the research to pursue "the combination of the focused component" in many components included in ginsenoside, in which 40 kinds or more have been currently known, and " the amount rate of the focused component", and set out the following object. That is, the object is to provide a ginsenoside composition, which can obtain an excellently, specifically result in terms of efficacy for various symptoms and diseases, including unidentified complaints.

### -2-

Furthermore, the present invention has the object to provide a ginsenoside composition which has excellent improvement effect for each symptoms and disease, can extend the width of symptoms and diseases, which can be improved, and can exert the maximum effect by a small amount of ingestion.

### Means for Solving the Problems

A ginsenoside composition comprising 3 components of Rb 1, Rg 3 (S), and Rg 2 (S) as essential components, wherein the Rb 1, the Rg 3 (S), and the Rg 2 (S) are ginsenosides, wherein, in solid base of the composition, a content of the Rb 1 is 7.3 mg/g or more, wherein a ratio of a content of the Rb 1 and a content of the Rg 3 (S), "Rb 1 / Rg 3 (S)", is in a range of 1.2 to 3.7, and wherein a ratio of a content of the Rg 3 (S) and a content of the Rg 2 (S), "Rg 3 (S) / Rg 2 (S)", is in a range of 1.7 to 4.4

### Effect of the Invention

### -1-

According to the present invention, when all of the following 3 requirements are fulfilled, a remarkably superior effect is exerted for each symptom in indefinite complaint or other symptoms of failing health.
- Requirement 1:
   "an amount ratio of Rb 1" is in the above specified range.
- Requirement 2:
   "Rb 1 / Rg 3(S)" which is the ratio of the content of the Rb 1 and the content of the above Rg 3 (S) is in the above specified range.
- Requirement 3:
   further, " Rg3 (S) / Rg2(S)" which is the ratio of the content of the Rg3 (S) and the content of the Rg2 (S) is in the above specified range.

### -2-

The typical examples of symptoms of the above "indefinite complaint" are fatigue, appetite, insomnia, cold hands and feet, bowel movements, motivation fullness, low back pain, stiff neck, stomach pain, abdominal bloating, sweating, stiffness of muscles or joints, and the like.

However, efficacies for symptomatic persons having each symptom in indefinite complaint when ingesting the ginsenoside component of the present invention, are extremely satisfactory.

### -3-

It is supposed that the fact, in which the above excellent result can be obtained when ingesting the ginsenoside component of the present invention, is the result of the complex action affected intertwined. The complex action is the response of the amount ratio and amount balance of the specified components "Rb 1, Rg 3 (S), and Rg2 (S)" which belong to diol-based ginsenoside and triol-based ginsenoside.

### Brief Explanation of Drawings

Fig. 1 is a scatter diagrams illustrating the relationship of "content of Rb 1" and "ratio of Rb 1 / Rg 3 (S)" about commercial ginsenoside preparations.
Fig. 2 is a scatter diagrams illustrating the relationship of "content of Rb1" and "ratio of Rg3 (S) / Rg2 (S)" about commercial ginsenoside preparations.
Fig 3. is a diagram illustrating the result when the tablets T 01 and T 02 are ingested by A group.
Fig. 4 is a diagram illustrating the result when the tablets T 03 and T 04 are ingested by B group.
Fig. 5 is a diagram illustrating the result when the tablets T 05 and T 06 are ingested by C group.
Fig. 6 is a diagram illustrating the result when the tablets T 07 and T 08 are ingested by D group.
Fig. 7 is a diagram illustrating the result when the tablets T 09 and T 10 are ingested by E group.
Fig. 8 is a diagram illustrating the result when the tablets T 11 and T 12 are ingested by F group.
Fig. 9 is a diagram illustrating the result when the tablets T 13 and T 14 are ingested by G group.
Fig. 10 is a diagram illustrating the result when the tablets T 15 and T 16 are ingested by H group.

### Detailed Description of the Preferred Embodiment

The present invention will be described in detail as follows.

### (4 requirements)

As described above, the ginsenoside compositions of the present invention is characterized by fulfilling all of the following requirements. In addition, the content below is a solid base of the composition.

The first requirement: The composition includes 3 components, i.e., Rb 1, Rg 3 (S), and Rg 2 (S) as essential components. (In addition, the contents and ratios of each component of ginsenosides which are other than the 3 components being the essential component not particularly limited. These components are shown in Table 2.)

The second requirement : The content of the above R b 1 is 7.3 mg/g or more. (Especially preferably, the content is in a range 7.3 to 13.0 mg/g.)

The third requirement : The ratio of the above content of Rb 1 and the content above Rg 3 (S), i.e., "Rb 1 / Rg 3 (S)", is in a range of 1.2 to 3.7.

The forth requirement: The ratio of the above content of Rg 3 (S) and the above content of Rg 2 (S), i.e., "Rg 3 (S) / Rg 2 (S)", is in a range of 1.7 to 4.4.

### (Supply source of each ginsenoside)

As the most preferable supply source of each ginsenoside is Korean ginseng, Korean red ginseng from Korean ginseng, an extract from Korean red ginseng, or powder of those. In addition to Korean ginseng, the ginsengs, such as Panax Notoginseng, American ginseng, Panax Japonicus and the like, these extracts, or these powders can be used.

### (Preparation of ginsenoside composition)

For preparing the ginsenoside composition of the present invention, a method of heating or fermenting mainly a root of ginseng or an extract of the root, and suitably adjusting the contents of Rb 1, Rg 3 (S), and Rg 2 (S), is adopted. Further, a method of mixing Rb 1, Rg 3 (S), and Rg 2 (S) isolated respectively is also adopted.

Further, the amount of each component can be also prepared by blending for the compositions obtained by the later method to the compositions obtained by the former method.

### (Quantification method of Rb 1, Rg 3 (S), Rg 2 (S))

### -1-

Although the quantification method of ginsenoside Rb 1, Rg 3 (S), Rg 2 (S) is not limited, in the present invention, the following method is adopted as a standard method.

### -2-

(a) : Ginsenoside component is extracted by accurately weighing 1 g of the sample powder and using the method described in "NINZIN" or "KOUZIN" in Japanese Pharmacopoeia.
(b) : Further, after roughly purifying the ginsenoside component using C18 simple column, the obtained component is filtered by a membrane filter and then made to be a sample solution.
(c) : Then, the sample solution is analyzed and quantity analyzed by high-performance liquid chromatography (HPLC) using C18 column.

### -3-

When ginsenoside Rb 1 and Rg 2 (S) are quantified, the sample is isolated by HPLC using a mobile phase of 30 to 35% acetonitrile concentration. The peak areas of ginsenoside Rb 1 and Rg 2 (S) of a standard sample and the peak areas of Rb 1 and Rg 2 (S) of the sample solution are compared, and then the contents of each ginsenoside in the sample are calculated.

### -4-

When ginsenoside Rg 3 (S) is quantified , the sample is isolated by HPLC using a mobile phase of 40 to 45% acetonitrile concentration. The peak area of ginsenoside Rg 3(S) of a standard sample and the peak area of Rg 3(S) of the sample solution are compared, and then the content of ginsenoside Rg 3 (S) in the sample is calculated.

### (Dosage form and uses of the composition)

The form of the composition (i.e., dosage form) of the present invention is, for example, a tablet (a disk form, a modified disk form, a square disk form, a ball, etc.), a granule, powders or extract powders, capsules, a solution, or the like. Here, the "extract powders" is made by powderizing the extract liquid using the method, for example, spray drying, or the like. When the tablets are made, an excipient can be used arbitrarily.

The representative example of the use of the composition of the present invention is an functional food such as health foods and health supplements. As the other use, various productions, such as, a general processed food, medical supplies, cosmetics, and bathe additives can be made.

### (Adaptation symptoms)

The composition of the present invention exerts an effect for indefinite complaint especially as described exemplary embodiments below. However, there are many cases exerting an effect to symptoms other than the indefinite complaint.

As described in "Background art", in the indefinite complaint, there are many symptoms, such as, fatigue, loss of appetite, insomnia, cold hands and feet, bowel movement disorders, lack of motivation fullness, low back pain, stiff neck, stomach pain, abdominal bloating, sweating, muscle and joint stiffness, etc. Thus, if there is only one symptom in these symptoms, ingestion is recommended.

As for the side effect of the composition, if it is not extremely overdosed outside the common sense, it is safe for an human body. This is the same as the case of general foods.

### [Ingestion amount]

Although the intake of the present invention is not limited, it is preferable to ingest 0.3 g or more daily. The standard ingestion is 1 to 6 g daily.

### Exemplary Embodiment

Then, the present invention will be further described according to the exemplary embodiments. In following, "%" is weight % except bricks %.

### [Exemplary embodiment 1]

### (Preparation of a tablet of the ginsenoside composition)

### -1-

Roots of Korean red ginseng having various values of Rb 1 and Re were pulverized respectively and an alcohol water containing ratio of water/ethanol of 4/6 in weight % were added to each pulverized ones by 10 times in weight. The ginsengs were extracted by stirring for 5 hours at 70 °C. Then, supernatant solutions were obtained by centrifuging, and concentrated to 40% bricks. The concentrated extracts were aged for a few days at 80 °C.

### -2-

In the aging period, middle sampling was carried out a few dozen times, and extract samples containing each element of Rb 1, Rg 3 (S) and Rg2 (S) in various concentrations are prepared.

### -3-

Then, these extract samples were powderized by a spray drying method, so that the powders containing ginsenoside having various component concentrations can be obtained.

### -4-

Then, tableting was carried out using these powders. The shape of the tablet was a circular disk form. The composition of the tablet was the 94% powder containing ginsenoside and 6% sucrose fatty acid esters which was excipients.

Each tablet contains 300mg.

### -5-

The 16 kinds tablets (T 01 to T 16) shown in Table 3 were made, where the contents of each component of ginsenosides were different.

In Table 3, the meanings of 1, 2 and 3 are as follows. In addition, in the signs in the column of compositions 1,2, and 3, ○ is in a range of the present invention, and × is out of range of the present invention.
·composition 1 : content of Rb 1(mg/g)
·composition 2 : ratio "content of Rb 1/content of Rg 3 (S)"
·composition 3 : ratio "content of Rb 3 (S) /content of Rg 2 (S)"

**Table 3**

| Tablet No. | Composition 1 | Composition 2 | Composition 3 |
|---|---|---|---|
| T 01 | × 5.6 | ○ 1.3 | ○ 3.7 |
| T 02 | ○ 14.3 | ○ 2.6 | ○ 2.1 |
| T 03 | ○ 8.4 | ○ 1.9 | ○ 2.0 |
| T 04 | ○ 14.6 | × 5.8 | × 1.0 |
| T 05 | × 5.3 | × 0.9 | ○ 2.2 |
| T 06 | ○ 12.3 | ○ 1.7 | ○ 4.3 |
| T 07 | ○ 14.3 | ○ 3.0 | ○ 3.9 |
| T 08 | ○ 11.9 | ○ 3.1 | ○ 1.9 |
| T 09 | ○ 12.0 | ○ 1.5 | × 5.1 |
| T 10 | ○ 10.4 | ○ 1.5 | ○ 3.0 |
| T 11 | × 4.9 | × 0.8 | × 4.7 |
| T 12 | ○ 7.8 | ○ 1.7 | × 1.0 |
| T 13 | ○ 8.3 | ○ 2.4 | × 5.0 |
| T 14 | ○ 15.0 | × 5.2 | × 4.8 |
| T 15 | ○ 8.4 | ○ 1.8 | ○ 4.2 |
| T 16 | ○ 14.5 | ○ 3.6 | ○ 3.6 |

### (Clinical trial)

### -1-

Using the above tablets T 01 to T 16, the clinical trials were carried out for 80 subjects having plural indefinite complaints. The breakdown of 80 people was men and women in each of 40 people. The average age of men was 63 and the average age of women is 62.

10 people (5 men and 5 women) were randomly divided respectively to 8 groups, i.e., A to H, and tablet ingestion tests were carried out without announcing the content of the tablet at all for the subjects excepting that the tablet was Korean ginseng.

### -2-

10 people in A group ingested the tablet "T 01" by 10 grains daily for 4 weeks, and after such period, 8 weeks washout period was set.

In addition, in the washout period, the people never ingested foods containing ginsenoside and made to return the condition having initial indefinite complaints. However, the number of symptom cases might be slightly different from the initial condition.

Then, the people ingested the tablet "T 02" by 10 grains daily for 4 weeks similarly in the first half. That is, for 10 people in A group, the clinical trial was " ingesting the tablet T 01 for 4 weeks in the first half", " washout period for 8 weeks" and "ingesting the tablet T 02 for 4 weeks in the second half.

### -3-

In above two ingesting tests in the first half and the second half, the people in A group were asked to fill the symptom in the subjective symptoms table below. That is, the starting time in the first half, the time immediately after to ingest for 4 weeks in the first half, the staring time in the second half, and the time immediately after to ingest for 4 weeks in the second half. The conditions for each symptom were grasped at each time.

### -4-

In the subjective symptoms table, the degree for each symptom was divided in 7 stages, and a person filling a number of 4 or less at the starting time was determined as "symptomatic cases".

Then, in the symptomatic cases, when the numbers at the time after 4 weeks in the first half and at the time after 4 weeks in the second half increase comparing with the starting time respectively, it was determined that there was an effect for the symptom. The efficacy ratio was calculated from the numbers of the people.

### -5-

Similarly, about B to H groups, the following ingesting tests were also carried out.
- 10 people in B group: " ingesting the tablet T 03 for 4 weeks in the first half", "washout period for 8 weeks", and "ingesting the tablet T 04 for 4 weeks in the second half.
- 10 people in C group: " ingesting the tablet T 05 for 4 weeks in the first half", " washout period for 8 weeks", and "ingesting the tablet T 06 for 4 weeks in the second half.
- 10 people in D group: " ingesting the tablet T 07 for 4 weeks in the first half", " washout period for 8 weeks", and "ingesting the tablet T 08 for 4 weeks in the second half.
- 10 people in E group: " ingesting the tablet T 09 for 4 weeks in the first half", " washout period for 8 weeks", and "ingesting the tablet T 10 for 4 weeks in the second half.
- 10 people in F group: " ingesting the tablet T 11 for 4 weeks in the first half", " washout period for 8 weeks", and "ingesting the tablet T 12 for 4 weeks in the second half.
- 10 people in G group: " ingesting the tablet T 13 for 4 weeks in the first half", " washout period for 8 weeks", and "ingesting the tablet T 14 for 4 weeks in the second half.
- 10 people in H group: " ingesting the tablet T 15 for 4 weeks in the first half", " washout period for 8 weeks", and "ingesting the tablet T 16 for 4 weeks in the second half.

(Items of the subjective symptoms table)

### -1-

In the beginning of the subjective symptoms table delivered to each people, the entry column of the name and the entry column of the tablet symbol are provided. Further, the following instruction and explanation are attached.

"Before ingesting the tablets (starting) and after 4 weeks, please fill the numbers that apply to each symptom by yourself. (If the number increases, health has increased)."

### -2-

Further, in the subjective symptoms table, 12 symptoms columns of A to L are provided. On the right side of the each symptoms column, blank columns to be written about "starting" and "4 weeks after" are provided. Thus, the subjects can fill the number corresponding to the symptom in the above symptom columns of A to L, at the starting time and 4 weeks after.

### -3-

### A. Fatigue

1. Fatigue is strong and you are bedridden and cannot do anything.
2. Fatigue is strong and you can do only personal thing.
3. There is fatigue, but you can return good when you rest about half a day.
4. There is fatigue, but it never lasts for hours.
5. There is fatigue, but you almost forget if working.
6. Fatigue is little.
7. There is no fatigue and you are completely healthy.

### -4-

### B. Appetite

1. You do not want anything to eat.
2. You cannot eat even when you are going to try to eat.
3. You are eating small amount choosing what you like.
4. You eat about half of meal given.
5. You can eat meal given and do not leave.
6. You cannot wait time of meal.
7. You eat something other than meals.

### -5-

### C. Insomnia

1. You often take sleeping pills, since you cannot sleep at night.
2. Falling sleep is hard, sleep is even light, and you wake up easily.
3. During the day, you snooze asleep. But you cannot sleep enough at night.
4. Falling sleep is good but you wake up easily by small sound or human voice.
5. Although you feel that falling sleep is hard, you imperceptibly sleep when you remain such a state.
6. You can sleep well after doing lightly something.
7. You think sleeping willingly.

### -6-

### D. Cold hands and feet (Please apply the current state to winter season.)

1. Since you get up at night for urination by cold of hands and feet , you cannot sleep well without electric blankets or heating pads.
2. Although you feel cold of hands and feet, it is not necessary to use electric brackets or heating pads in a bedding.
3. You prefer not to leave from sunny location or a place where there is a heater, a kotatsu, a stove.
4. You do not feel the necessity of heaters, but overdress.
5. You become warm by moving your body a little, when you feel to be cold of hands and feet.
6. You do not feel cold of hands and feet.
7. You feel that your body is warm and also to be warm of hands and feet.

### -7-

### E. Bowel movement

1. You are in constipation and cannot defecate without the help of laxatives and enemas in many time.
2. It is not necessary to use laxatives and enemas. However, there are a few days not defecating.
3. You repeat constipation and diarrhea sometimes, but can tentatively defecate every day.
4. You can defecate every day, but the time is irregular.
5. Although there are constipation and diarrhea rarely, you can defecate regularly in every day.
6. You can defecate regularly once a day.
7. There is defecation more than once a day and sometimes in loose feces.

### -8-

### F. Motivation fullness

1. There is no motivation for all things.
2. You do things that you like, but there is no motivation for the other things.
3. You do reluctantly things other than that you like.
4. You do things other than that you like, because there is no way.
5. There is a little motivation for the things other than that you like.
6. you have motivation for all things.
7. You have strong motivation for all things.

### -9-

### G. Low back pain

1. You cannot get up since you feel that your waist is heavy and pain. You want to lay.
2. Although you feel that your waist is heavy and pain, you can do personal things by yourself.
3. Although you feel sometimes that your waist is pain, there is no disturbance to do light work.
4. Sometimes you feel that your waist is heavy and dull pain, but it becomes better by massaging.
5. Although you feel that your waist is heavy and dull pain, you can forget it if you do something earnestly.
6. Sometimes you feel that your waist is dull pain, the pain disappears naturally if left alone.
7. You do not feel the waist pain at all.

### -10-

### H. Stiff neck

1. The stiff of shoulder and neck is hard, you cannot move body by nausea and dizziness.
2. The shoulder and neck are stiffened but it becomes better by massaging. You can do personal things by yourself.
3. The shoulder and neck are stiffened and pain, but you can do work if forcibly.
4. Although you are anxious for the stiff of shoulder and neck, you often forget when you concentrate on things.
5. The shoulder and neck are stiffened, but the degree is not so large so that you leave as it is.
6. The shoulder and neck are stiffened lightly. However, you are healable naturally by leaving as it is.
7. The shoulder and neck is not stiffened. You feel always that your body is light.

### -11-

### I. Stomach pain

1. Stomach is always ponderous and sometimes aching, so that there is a time to take the medicine of stomach.
2. There is a time that the stomach is ponderous and aching, but you endure in the quiet.
3. Sometimes, there is a time that the stomach is ponderous and aching, but the degree is not enough to endure.
4. Sometimes, you are concerned about the stomach pain, but you have forgotten by absorbing in something.
5. Sometimes, you feel the stomach pain, but you can be healed naturally by leaving alone.
6. You never feel stomach pain almost.
7. You do not mind stomach at all.

### -12-

### J. Abdominal bloating

1. The belly is bloated and you cannot breath.
2. The belly is bloated like that the stomach is pushed up, and burp comes out.
3. Gas is filed in the belly to sound of "Guru Guru" , so that you feel heavily.
4. Gas is bloated in the belly and you feel uncomfortable.
5. The belly is bloated and you feel uncomfortable. But gas go out well.
6. The belly is not bloated but gas go out well.
7. The belly condition is good and you feel refreshing.

### -13-

### K. Sweating

1. Even when you exercise vigorously, you almost never sweat.
2. You do not fairly sweat than normal people.
3. You do not sweat than normal people.
4. You feel that sweating is normal.
5. You feel that you sweat a little.
6. You feel that you sweat much.
7. You sweat very much when you work a little.

### L. Muscle and joint stiffness

1. Muscle and joint stiffen very much and you cannot move your body.
2. Muscle and joint stiffen and you feel pain. But you can work if you move forcibly.
3. Muscle and joint stiffen and you feel pain a little. But you can work if you move forcibly a little.
4. Muscle and joint stiffen and you feel anxious. But you can forget if you earnestly do things.
5. Muscle and joint stiffen sometimes. But you do not feel anxious almost.
6. Muscle and joint stiffen sometimes. But you do not feel anxious at all.
7. Muscle and joint do not stiffen and you feel always that your body is light.

### (the results of the ingesting tests)

### -1-

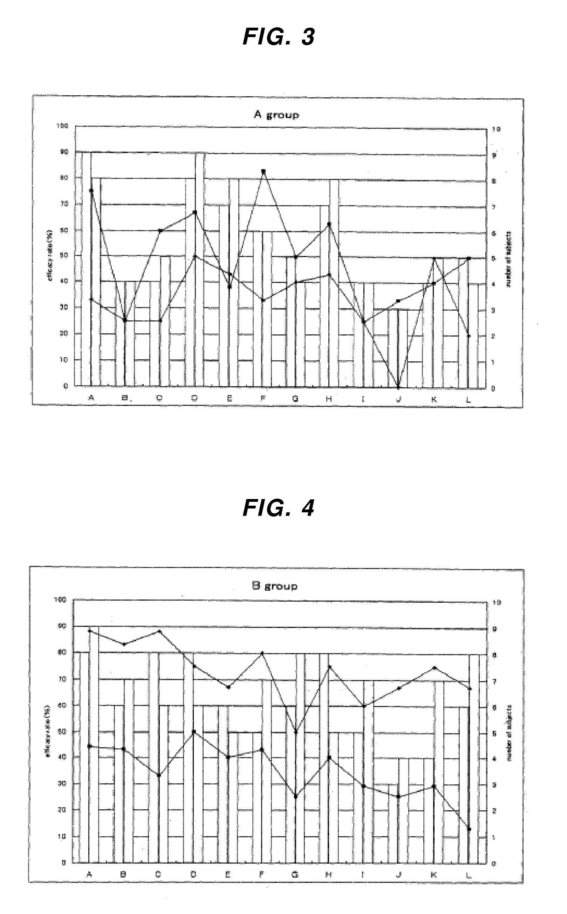

Fig. 3 and Table 4 illustrate the results when the A group ingests tablets T 01 and T 02.

In Fig. 3, the bar graph on left side is the number of subjects about the tablet T 01, and the bar graph on the right side is the number of subjects about the tablet T 02.

The line graph in Fig. 3, the plots of ◆ mark are the efficacy ratio (%) about tablet T 01, and the plots of ■ mark are the efficacy ratio about tablet T 02.

### -2-

In B group to H group (Fig. 4 to Fig. 10, and Table 5 to Table 11), the above descriptions are similarly applied.

Fig. 4 and Table 5 are the results when the B group ingests tablets T 03 and T 04.

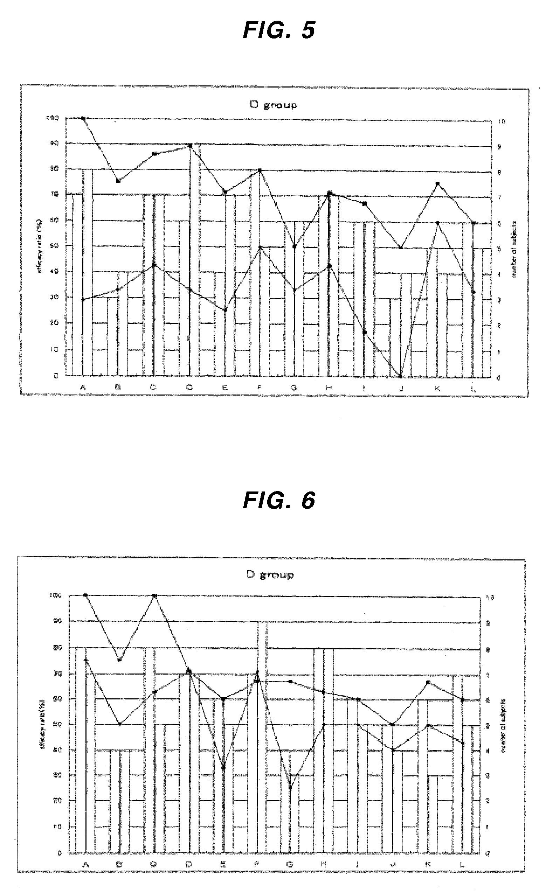

Fig. 5 and Table 6 are the results when the C group ingests tablets T 05 and T 06.

Fig. 6 and Table 7 are the results when the D group ingests tablets T 07 and T 08.

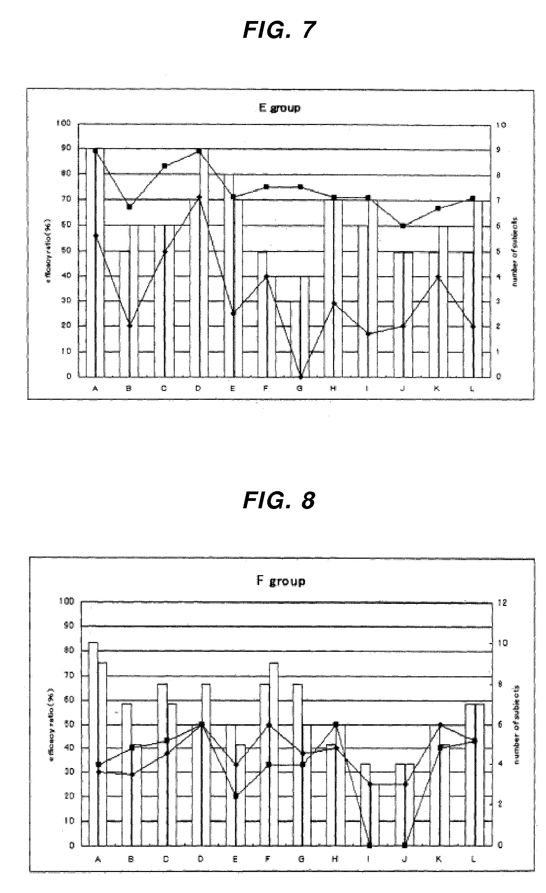

Fig. 7 and Table 8 are the results when the E group ingests tablets T 09 and T 10.

Fig. 8 and Table 9 are the results when the F group ingests tablets T 11 and T 12.

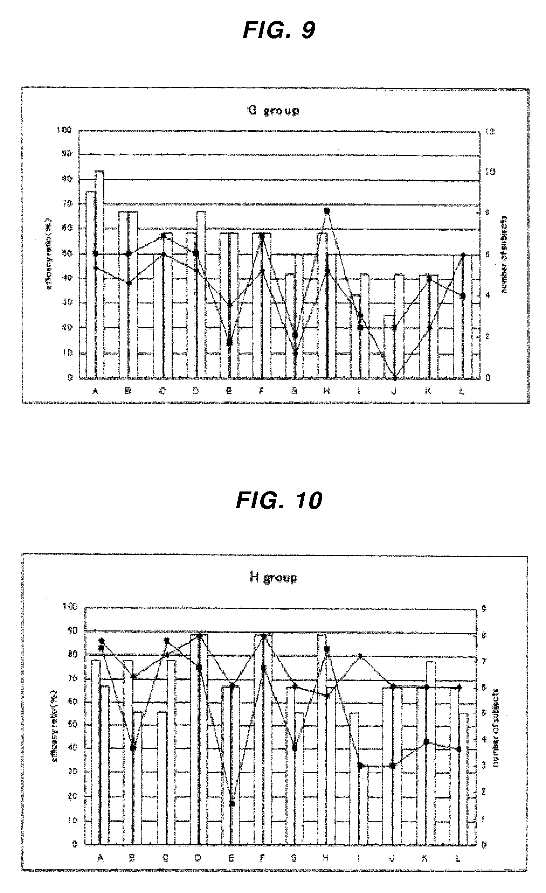

Fig. 9 and Table 10 are the results when the G group ingests tablets T 13 and T 14.

Fig. 10 and Table 11 are the results when the H group ingests tablets T 15 and T 16.

**[Table 4]**

| A Group | Left bar graph Number of subjects of Tablet T01 (people) | Right bar graph Number of subjects of Tablet T02 (people) | ◆ in line graph: Efficacy ratio of Tablet T01 (%) | ■ in line graph: Efficacy ratio of Tablet T02 (%) |
|---|---|---|---|---|
| Fatigue | 9 people | 8 people | 33% | 75% |
| Appetite | 4 people | 4 people | 25% | 25% |
| Insomnia | 4 people | 5 people | 25% | 60% |
| Cold hands and feet | 8 people | 9 people | 50% | 67% |
| Bowel movement | 7 people | 8 people | 43% | 38% |
| Motivation fullness | 6 people | 6 people | 33% | 83% |
| Low back pain | 5 people | 4 people | 40% | 50% |
| Stiff neck | 7 people | 8 people | 43% | 63% |
| Stomach pain | 4 people | 4 people | 25% | 25% |
| Abdominal bloating | 3 people | 3 people | 0% | 33% |
| Sweating | 4 people | 5 people | 50% | 40% |
| Muscle and joint stiffness | 5 people | 4 people | 20% | 50% |

**[Table 5]**

| B Group | Left bar graph Number of Subjects of Tablet T03 (people) | Right bar graph Number of subjects of Tablet T04 (people) | ◆ in line graph: Efficacy ratio of Tablet T03 (%) | ■ in line graph: Efficacy ratio of Tablet T04 (%) |
|---|---|---|---|---|
| Fatigue | 9 people | 9 people | 88% | 44% |
| Appetite | 6 people | 7 people | 83% | 43% |
| Insomnia | 8 people | 6 people | 88% | 33% |
| Cold hands and feet | 8 people | 6 people | 75% | 50% |
| Bowel movement | 6 people | 5 people | 67% | 40% |
| Motivation fullness | 5 people | 7 people | 80% | 43% |
| Low back pain | 6 people | 8 people | 50% | 25% |
| Stiff neck | 8 people | 5 people | 75% | 40% |
| Stomach pain | 5 people | 7 people | 60% | 29% |
| Abdominal bloating | 3 people | 4 people | 67% | 25% |
| Sweating | 4 people | 7 people | 75% | 29% |
| Muscle and joint stiffness | 6 people | 8 people | 67% | 13% |

**[Table 6]**

| C Group | Left bar graph Number of subjects of Tablet T05 (people) | Right bar graph Number of subjects of Tablet T06 (people) | ◆ in line graph: Efficacy ratio of Tablet T05 (%) | ■ in line graph: Efficacy ratio of Tablet T06 (%) |
|---|---|---|---|---|
| Fatigue | 7 people | 8 people | 29% | 100% |
| Appetite | 3 people | 4 people | 33% | 75% |
| Insomnia | 7 people | 7 people | 43% | 86% |
| Cold hands and feet | 6 people | 9 people | 33% | 89% |
| Bowel movement | 4 people | 7 people | 25% | 71% |
| Motivation fullness | 8 people | 5 people | 50% | 80% |
| Low back pain | 6 people | 6 people | 33% | 50% |
| Stiff neck | 7 people | 7 people | 43% | 71% |
| Stomach pain | 6 people | 6 people | 17% | 67% |
| Abdominal bloating | 3 people | 4 people | 0% | 50% |
| Sweating | 5 people | 4 people | 60% | 75% |
| Muscle and joint stiffness | 6 people | 5 people | 33% | 60% |

**[Table 7]**

| D Group | Left bar graph Number of subjects of Tablet T07 (people) | Right bar graph Number of subjects of Tablet T08 (people) | ◆ in line graph: Efficacy ratio of Tablet T07 (%) | ■ in line graph: Efficacy ratio of Tablet T08 (%) |
|---|---|---|---|---|
| Fatigue | 8 people | 7 people | 75% | 100% |
| Appetite | 4 people | 4 people | 50% | 75% |
| Insomnia | 8 people | 5 people | 43% | 100% |
| Cold hands and feet | 7 people | 7 people | 63% | 71% |
| Bowel movement | 6 people | 5 people | 71% | 60% |
| Motivation fullness | 7 people | 9 people | 33% | 67% |
| Low back pain | 4 people | 3 people | 71% | 67% |
| Stiff neck | 8 people | 8 people | 25% | 63% |
| Stomach pain | 6 people | 5 people | 50% | 60% |
| Abdominal bloating | 5 people | 4 people | 40% | 50% |
| Sweating | 6 people | 3 people | 50% | 67% |
| Muscle and joint stiffness | 7 people | 5 people | 43% | 60% |

**[Table 8]**

| E Group | Left bar graph Number of subjects of Tablet T09 (people) | Right bar graph Number of Subjects of Tablet T10 (people) | ◆ in line graph: Efficacy ratio of Tablet T09 (%) | ■ in line graph: Efficacy ratio of Tablet T10 (%) |
|---|---|---|---|---|
| Fatigue | 9 people | 9 people | 56% | 89% |
| Appetite | 5 people | 6 people | 20% | 67% |
| Insomnia | 6 people | 6 people | 50% | 83% |
| Cold hands and feet | 7 people | 9 people | 71% | 89% |
| Bowel movement | 8 people | 7 people | 25% | 71% |
| Motivation fullness | 5 people | 4 people | 40% | 75% |
| Low back pain | 3 people | 4 people | 0% | 75% |
| Stiff neck | 7 people | 7 people | 29% | 71% |
| Stomach pain | 6 people | 7 people | 17% | 71% |
| Abdominal bloating | 5 people | 5 people | 20% | 60% |
| Sweating | 5 people | 6 people | 40% | 67% |
| Muscle and joint stiffness | 5 people | 7 people | 20% | 71% |

**[Table 9]**

| F Group | Left bar graph Number of subjects of Tablet T11 (people) | Right bar graph Number of subjects of Tablet T12 (people) | ◆ in line graph: Efficacy ratio of Tablet T11 (%) | ■ in line graph: Efficacy ratio of Tablet T12 (%) |
|---|---|---|---|---|
| Fatigue | 10 people | 9 people | 30% | 33% |
| Appetite | 7 people | 5 people | 29% | 40% |
| Insomnia | 8 people | 7 people | 38% | 43% |
| Cold hands and feet | 6 people | 8 people | 50% | 50% |
| Bowel movement | 6 people | 5 people | 33% | 20% |
| Motivation fullness | 8 people | 9 people | 50% | 33% |
| Low back pain | 8 people | 6 people | 38% | 33% |
| Stiff neck | 5 people | 6 people | 40% | 50% |
| Stomach pain | 4 people | 3 people | 25% | 0% |
| Abdominal bloating | 4 people | 4 people | 25% | 0% |
| Sweating | 6 people | 5 people | 50% | 40% |
| Muscle and joint stiffness | 7 people | 7 people | 43% | 43% |

**[Table 10]**

| G group | Left bar graph Number of subjects of Tablet T13 (people) | Right bar graph Number of Subjects of Tablet T14 (people) | ◆ in line graph: Efficacy ratio of Tablet T13 (%) | ■ in line graph: Efficacy ratio of Tablet T14 (%) |
|---|---|---|---|---|
| Fatigue | 9 people | 10 people | 44% | 50% |
| Appetite | 8 people | 8 people | 38% | 50% |
| Insomnia | 6 people | 7 people | 50% | 57% |
| Cold hands and feet | 7 people | 8 people | 43% | 50% |
| Bowel movement | 7 people | 7 people | 29% | 14% |
| Motivation fullness | 7 people | 7 people | 43% | 57% |
| Low back pain | 5 people | 6 people | 10% | 17% |
| Stiff neck | 7 people | 6 people | 43% | 67% |
| Stomach pain | 4 people | 5 people | 25% | 20% |
| Abdominal bloating | 3 people | 5 people | 0% | 20% |
| Sweating | 5 people | 5 people | 20% | 40% |
| Muscle and joint stiffness | 6 people | 6 people | 50% | 33% |

**[Table 11]**

| H group | Left bar graph Number of subjects of Tablet T15 (people) | Right bar graph Number of subjects of Tablet T16 (people) | ◆ in line graph: Efficacy ratio of Tablet T15 (%) | ■ in line graph: Efficacy ratio of Tablet T16 (%) |
|---|---|---|---|---|
| Fatigue | 7 people | 6 people | 86% | 83% |
| Appetite | 7 people | 5 people | 71% | 40% |
| Insomnia | 5 people | 7 people | 80% | 86% |
| Cold hands hand feet | 8 people | 8 people | 88% | 75% |
| Bowel movement | 6 people | 6 people | 67% | 17% |
| Motivation fullness | 8 people | 8 people | 88% | 75% |
| Low back pain | 6 people | 5 people | 67% | 40% |
| Stiff neck | 8 people | 6 people | 63% | 83% |
| Stomach pain | 5 people | 3 people | 80% | 33% |
| Abdominal bloating | 6 people | 6 people | 67% | 33% |
| Sweating | 6 people | 7 people | 67% | 43% |
| Muscle and joint stiffness | 6 people | 5 people | 67% | 40% |

### (Summary of the results of the ingesting tests)

### -1-

"Tablets T 01 to T 16" used in these ingesting tests above, "the composition of the ginsenoside component of these tablets", and "the average efficacy ratio", are shown in Table 12.

### -2-

In Table 12, the column of the average efficacy ratio is added to the above Table 3 and 3 stage evaluations of "○, □, and Δ" are added. Further, in Table 12, each line is rearranged by the block order of "○, □, and Δ".

### -3-

The meanings of the marks in Table 12 are as follows.

In the marks in "composition" column, as similarly as above Table 4, ○ and □ are in claim 1 in the present invention and × is out of claim 1 in the present invention.

In the "average efficacy ratio" column, ○ is preferable, □ is next preferable, and Δ is slightly inferior. About the average efficacy ratios, it can be understood that the reason of the difference of the evaluations of the ○ group and the □ group is due to the fact that the composition 1 (amount of Rb 1) in the □ group is slightly excessive than the optimum range comparing with the ○ group.

### -4-

From Table 12, it can be understood that the preferable result is obtained when all of the following compositions 1, 2, and 3 are satisfied. "composition 1": the amount of Rb 1 is 7.3 mg/g or more. (especially, 7.3 to 13.0 mg/g)
"composition 2": the content ratio of Rb 1 / Rg 3 (S) is in the range of 1.2 to 3.7. "composition 3": the content ratio of Rg 3 (S) / Rg 2 (S) is in the range of 1.7 to 4.4 mg/g.

**[Table 12]**

| Tablet No. | Composition 1 | Composition 2 | Composition 3 | Average Efficacy ratio |
|---|---|---|---|---|
| T 03 | ○ 8.4 | ○ 1.9 | ○ 2.0 | ○ 73% |
| T 06 | ○ 12.3 | ○ 1.7 | ○ 4.3 | ○ 73% |
| T 08 | ○ 11.8 | ○ 3.1 | ○ 1.9 | ○ 70% |
| T 10 | ○ 10.4 | ○ 1.5 | ○ 3.0 | ○ 74% |
| T 15 | ○ 8.4 | ○ 1.8 | ○ 4.2 | ○ 74% |
| T 02 | ○ 14.3 | ○ 2.6 | ○ 2.1 | □ 51% |
| T 07 | ○ 14.3 | ○ 3.0 | ○ 3.9 | □ 52% |
| T 16 | ○ 14.5 | ○ 3.6 | ○ 3.6 | □ 54% |
| T 01 | × 5.6 | ○ 1.3 | ○ 3.7 | Δ 32% |
| T 04 | ○ 14.6 | × 5.8 | × 1.0 | Δ 35% |
| T 05 | × 5.3 | × 0.9 | ○ 2.2 | Δ 33% |
| T 09 | ○ 12.0 | ○ 1.5 | × 5.1 | Δ 32% |
| T 11 | × 4.9 | × 0.8 | × 4.7 | Δ 38% |
| T 12 | ○ 7.8 | ○ 1.7 | × 1.0 | Δ 32% |
| T 13 | ○ 8.3 | ○ 2.4 | × 5.0 | Δ 33% |
| T 14 | ○ 15.0 | × 5.2 | × 4.8 | Δ 40% |

### Industrial Applicability

The ginsenoside composition according to the present invention has a remarkable improvement for various symptoms and diseases including indefinite complaint. In addition to this, the ginsenoside composition can extend the width of the improvable symptoms and diseases. Further, since the ginsenoside composition can exert the maximum effect with a small amount of ingestion, the composition is especially useful as an oral composition for indefinite complaint improvement or nutritional fortification, including a functional food such as health foods and health supplements. Otherwise, the ginsenoside composition is also useful as general processed foods, medical products, cosmetics, and a bath agent.

## Claims

1. A ginsenoside composition comprising 3 components of Rb 1, Rg 3 (S), and Rg 2 (S) as essential components,
wherein the Rb 1, the Rg 3 (S), and the Rg 2 (S) are ginsenosides, wherein, in solid base of the composition, a content of the Rb 1 is 7.3 mg/g or more,
wherein a ratio of a content of the Rb 1 and a content of the Rg 3 (S), "Rb 1 / Rg 3 (S)", is in a range of 1.2 to 3.7, and
wherein a ratio of a content of the Rg 3 (S) and a content of the Rg 2 (S), "Rg 3 (S) / Rg 2 (S)", is in a range of 1.7 to 4.4

2. The ginsenoside composition according to claim 1,
wherein the ginsenoside composition is an oral composition for indefinite complaint improvement or nutritional fortification.
